# EUROPÄISCHE PATENTANMELDUNG

(11) **EP 2 060 283 A1**
(43) Veröffentlichungstag der Anmeldung: **20.05.2009**
(21) Anmeldenummer: 07021981.1
(22) Anmeldetag: 13.11.2007
(51) Int. Cl.: A61M 5/14

(54) **Vorrichtung zum Zusammenführen mehrerer Infusionen und/oder Injektionen**

(71) Anmelder: RoweMed AG - Medical 4 Life, 19370 Parchim (DE)
(72) Erfinder: Wex, Roland, 34212 Melsungen (DE)
(74) Vertreter: Quermann, Helmut

(57) **Zusammenfassung**

Die Erfindung betrifft eine Vorrichtung (1) zum Zusammenführen mehrerer Infusionen und/oder Injektionen, mit einem von der Infusions-/oder Injektionsflüssigkeit durchströmten Gehäuse (2), sowie mit einem mit dem Gehäuse (2) verbundenen Lagerteil (3), das im Bereich einer Seite (6) des Gehäuses (2) angeordnet ist, und das Gehäuse (2) im Bereich anderer Gehäuseseiten (7, 8, 27) mehrere, jeweils separat verschließbare Zugänge (9) für die Flüssigkeit und einen Abgang für die zusammengeführte Flüssigkeit aufweist.

Bei einer derartigen Vorrichtung ist erfindungsgemäß vorgesehen, dass das Lagerteil (3) eine nach innen gerichtete Aufnahme (16) aufweist, die dem teilweisen Umschließen eines Lagerstabes für die Vorrichtung (1) dient, sowie ein bandförmiges Befestigungsmittel (19) dem Umschließen der dem Lagerteil (3) abgewandten Seite des Lagerstabs dient, wobei das Befestigungsmittel (19) im Bereich eines Endes (26) mit dem Lagerteil (3) verbunden oder verbindbar ist und das Befestigungsmittel (19) im Bereich des anderen Endes (22) mit dem Lagerteil (3) verbindbar ist.

## Beschreibung

Die Erfindung betrifft eine Vorrichtung zum Zusammenführen mehrerer Infusionen und/oder Injektionen, mit einem von der Infusions-/Injektionsflüssigkeit durchströmten Gehäuse, sowie mit einem mit dem Gehäuse verbundenen Lagerteil, das im Bereich einer Seite des Gehäuses angeordnet ist, und das Gehäuse im Bereich anderer Gehäuseseiten mehrere jeweils separat verschließbare Zugänge für die Flüssigkeit und einen Abgang für die zusammengeführte Flüssigkeit aufweist.

Eine derartige Vorrichtung ist aus der DE 40 04 134 A1 bekannt. Bei dieser besteht das Gehäuse aus einem Gehäuseunterteil und einem mit diesem verbundenen Gehäuseoberteil. Das Gehäuse ist halbkreisförmig ausgebildet und weist auf seiner nicht gekrümmten Seite das Lagerteil auf. Das plattenförmige Lagerteil weist eine rechteckige obere Fläche auf. Das Lagerteil und das Gehäuseunterteil sind einteilig ausgebildet, wobei das Lagerteil als Platte gestaltet ist. Vor dem Gebrauch der Vorrichtung wird das plattenförmige Lagerteil, bei horizontaler Orientierung des Lagerteils und des Gehäuses, an einem Ständer fixiert. Der Abgang wird über eine Schlauchverbindung mit einem Patienten verbunden und, entsprechend der Anzahl der Infusionen, Infusionsschläuche mit den Zugängen verbunden. Aufgrund der Gestaltung der Zugänge als separat verschließbare Zugänge kann Flüssigkeit über den jeweiligen Zugang in Art eines Rückschlagventils in das Innere des Gehäuses gelangen und verlässt dieses durch das Filterelement und den Abgang. Gehäuse und Lagerteil bestehen beispielsweise aus einem thermoplastisch verformbaren, durchsichtigen Kunststoff, insbesondere PVC.

Aufgabe der vorliegenden Erfindung ist es, eine Vorrichtung der genannten Art so weiter zu bilden, dass diese, bei geringem baulichen Aufwand, ein sicheres und schnelles Befestigen ermöglicht.

Gelöst wird die Aufgabe dadurch, dass das Lagerteil eine nach innen gerichtete Aufnahme aufweist, die dem teilweisen Umschließen eines Lagerstabs für die Vorrichtung dient, sowie ein bandförmiges Befestigungsmittel dem Umschließen der dem Lagerteil abgewandten Seite des Lagerstabs dient, wobei ein Ende des Befestigungsmittel mit dem Lagerteil verbunden oder verbindbar ist und das andere Ende des Befestigungsmittel mit dem Lagerteil verbindbar ist.

Wesentlich ist bei der erfindungsgemäße Vorrichtung somit, dass der Infusions-/Injektionssammler, den die Vorrichtung darstellt, eine integrierte Befestigung an einem Stab aufweist. Bei diesem Stab handelt es sich insbesondere um ein Standardstativ. Dieses Standardstativ bzw. dieser Lagerstab weist insbesondere einen kreisförmigen Querschnitt auf.

Die Vorrichtung besitzt alle erforderlichen Bestandteile, um sie an einem Stab zu befestigen. Es ist nicht erforderlich, separate Befestigungsmittel am Stab vorzusehen. Die Gestaltung der Vorrichtung ermöglicht es dem Personal, bei einfacher Handhabung die Vorrichtung sicher und schnell zu befestigen. Es ist nur erforderlich, das Lagerteil mit seiner Aufnahme auf den Stab hin auszurichten, d.h. die Aufnahme des Lagerteils so zum Stab zu positionieren, dass der Stab aufgrund der nach innen gerichteten Geometrie der Aufnahme von dieser teilweise umschlossen wird. Anschließend ist es nur noch erforderlich, das bandförmige Befestigungsmittel mit dem Lagerteil zu verbinden. Das bandförmige Befestigungsmittel ist insbesondere elastisch oder als Klettband ausgebildet. Derartige Befestigungsmittel lassen sich besonders einfach und preisgünstig herstellen.

Vorzugsweise ist die nach innen gerichtete Aufnahme des Lagerteils bogenförmig gestaltet, insbesondere annähernd kreisbogenförmig gestaltet. Die Bogenform und die Abmessungen des Bogens sind auf den Querschnitt, insbesondere den Durchmesser des Lagerstabs abgestimmt, so dass die Aufnahme über einen ausreichenden Bogenabschnitt oder aber über ausreichende Bogenabschnitte den am Lagerteil anliegenden Lagerstab kontaktiert. Bei am Lagerteil befestigtem Befestigungsmittel, das den Lagerstab umschließt, ist das Befestigungsmittel vorzugsweise geringfügig gespannt, insbesondere aufgrund dessen Elastizität. Hierdurch ist eine sichere Befestigung der Vorrichtung am Lagerstab bzw. Standardstativ gewährleistet.

Die Anordnung des Befestigungsmittels am Lagerteil kann auf unterschiedliche Art und Weise erfolgen. So kann das Befestigungsmittel im Bereich eines Endes fest mit dem Lagerteil verbunden sein, somit unlösbar mit dem Lagerteil verbunden sein. Es ist demnach beim Anbringen der Vorrichtung am Lagerstab nur erforderlich, das andere Ende des Befestigungsmittels hinter dem Lagerstab vorbeizuführen und dieses andere Ende auf der dem einen Ende des Befestigungsmittels abgewandten Seite des Lagerteils mit diesem zu verbinden. - Es ist genauso möglich, das Befestigungsmittel und entsprechende Aufnahmen des Lagerteils so zu gestalten, dass das Befestigungsmittel im Bereich beider Enden lose ist und zum Anbringen der Vorrichtung mit dem Lagerteil zu verbinden ist. Die Variante der dauerhaften Befestigung des Befestigungsmittels im Bereich eines Endes mit dem Lagerteil ist aber unter dem Aspekt der Verliersicherheit des Befestigungsmittels von Vorteil. Dies erhöht die Sicherheit des Handlings der Vorrichtung.

Es wird als vorteilhaft angesehen, wenn das Befestigungsmittel, insbesondere das elastische Band, zumindest im Bereich eines Endes mindestens ein Loch aufweist, sowie das Lagerteil in seinem mit diesem Ende des Befestigungsmittels zusammenwirkenden Bereich mit einem Vorsprung versehen ist, wobei bei mit dem Lagerteil befestigten, den Lagerstab umschließenden Befestigungsmittel der Vorsprung das Loch durchsetzt und das Befestigungsmittel vorgespannt ist. In aller Regel ist es ausreichend, ein Befestigungsmittel bzw. Band vorzusehen, das nur ein Loch aufweist, da aufgrund der Elastizität des Befestigungsmittels die Vorrichtung an Lagerstäben mit unterschiedlichem Durchmesser angebracht werden kann. Zweckmäßiger ist es allerdings, zumindest zwei Löcher am Ende des Befestigungsmittels vorzusehen, so dass, je nach Durchmesser des Lagerstabs, das eine oder andere Loch mit dem Vorsprung des Lagerteils zusammenwirkt. Das Band weist insbesondere im Bereich eines Endes mehrere, vorzugsweise zwei in Längsrichtung des Bandes hintereinander angeordnete Löcher auf.

Gemäß einer besonderen Ausführungsform der Erfindung ist vorgesehen, dass das Gehäuse flach ausgebildet ist. Hierdurch wird ein relativ kleines Verhältnis zwischen Höhe des Gehäuses, ohne den ebenfalls unten vorstehenden Abgang betrachtet, zu den Längen-/Breitenabmessungen des Gehäuses verstanden. Bei diesem flach ausgebildeten Gehäuse ist das Lagerteil vorzugsweise mit einer Seite des Gehäuses verbunden, die eine geringe Höhe aufweist.

Es wird unter dem Aspekt der Handhabung der Vorrichtung als vorteilhaft angesehen, wenn das Gehäuse im wesentlichen die Form eines gleichschenkligen Dreiecks oder eines gleichschenkliges Trapezes aufweist, wobei das Lagerteil im Bereich der Grundseite von Dreieck bzw. Trapez angeordnet ist. Die Grundseite des Dreiecks ist diejenige, von der die anderen Schenkel des Dreiecks unter demselben Winkel abgehen. Die Grundseite des Trapezes ist vorzugsweise die längere der parallelen Trapezseiten. Die Zugänge der Vorrichtung sind zweckmäßig im Bereich der anderen Seite des Dreiecks bzw. Trapezes angeordnet. Für das Dreieck bedeutet dies, dass die Zugänge im Bereich der beiden anderen Seiten des Dreiecks angeordnet sind, für das Trapez bedeutet dies, dass die Zugänge im Bereich der drei anderen Seiten des Trapezes angeordnet sind. Gegebenenfalls kann die andere Grundseite geringfügig geknickt ausgebildet sein, mit einer Wölbung von der Grundseite des Trapezes weg gerichtet.

Bei Ausbildung des Gehäuses als Dreieck weist die jeweilige der beiden mit Zugängen versehenen Seiten des Gehäuses vorzugsweise zwei Zugänge auf. Bei Ausbildung des Gehäuses als Trapez weist jede der drei Seiten des Trapezes, die die Zugänge aufweisen, zwei Zugänge auf.

Der Abgang ist insbesondere auf der Unterseite des Gehäuses angeordnet.

Konstruktiv ist die Vorrichtung insbesondere derart gestaltet, dass das Gehäuse zumindest zweiteilig ausgebildet ist und ein oberes und ein unteres Gehäuseteil aufweist, wobei eines der Gehäuseteile und das Lagerteil einteilig sind. Insbesondere das untere Gehäuseteil und das Lagerteil bilden ein Bauteil. Das Gehäuse und das Lagerteil bestehen zweckmäßig aus Kunststoff und sind als Spritzgussteile ausgebildet, die miteinander verschweißt sind.

Bei der Vorrichtung erfolgt das Zusammenführen der Flüssigkeit und das Ableiten der zusammengeführten Flüssigkeit in einem geschlossenen System. Im Gehäuse ist insbesondere ein Filterelement für die Flüssigkeit und/oder ein Filterelement für im Gehäuse befindliche Luft angeordnet.

Weitere Merkmale der Erfindung sind in der Beschreibung der Zeichnung und in den Unteransprüchen dargestellt, wobei bemerkt wird, dass alle Einzelmerkmale und alle Kombinationen von Einzelmerkmalen weitere erfinderische Ausgestaltungen darstellen.

In der Zeichnung ist die Erfindung anhand zweier Ausführungsformen beispielsweise dargestellt, ohne auf diese beschränkt zu sein. Es stellt dar:
- Figur 1: eine erste Ausführungsform der erfindungsgemäßen Vorrichtung in einer räumlichen Ansicht, schräg von oben gesehen,
- Figur 2: die Vorrichtung gemäß Figur 1 in einer räumlichen Anordnung, schräg von unten gesehen,
- Figur 3: die Vorrichtung gemäß der Figuren 1 und 2 in einer Seitenansicht gesehen,
- Figur 4: die Vorrichtung gemäß den Figuren 1 bis 3 in einer Draufsicht gesehen,
- Figur 5: die Vorrichtung gemäß der Figuren 1 bis 4 in einer Unteransicht gesehen,
- Figuren 6 bis 10: Ansichten der Vorrichtung gemäß der zweiten Ausführungsform entsprechend den Darstellungen in den Figuren 1 bis 5.

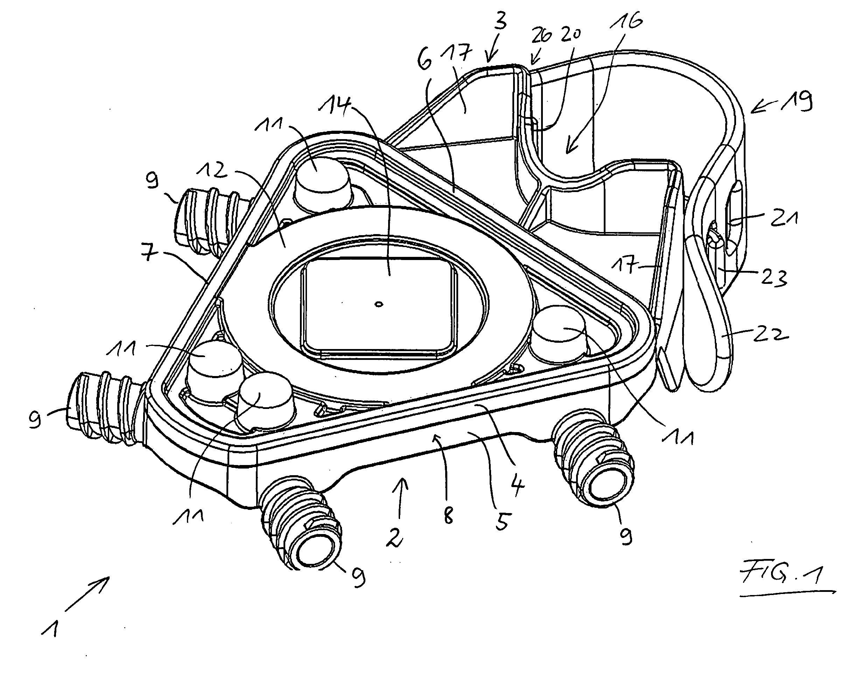

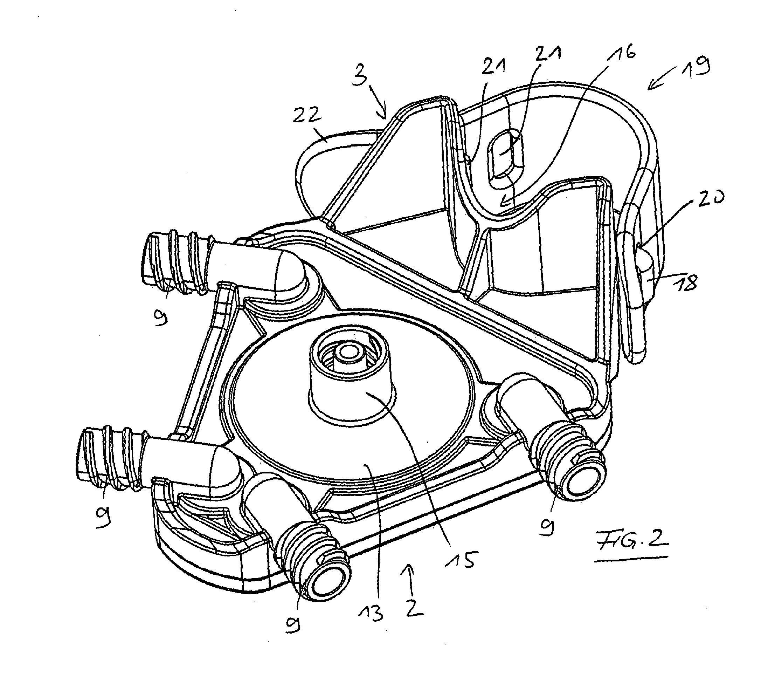

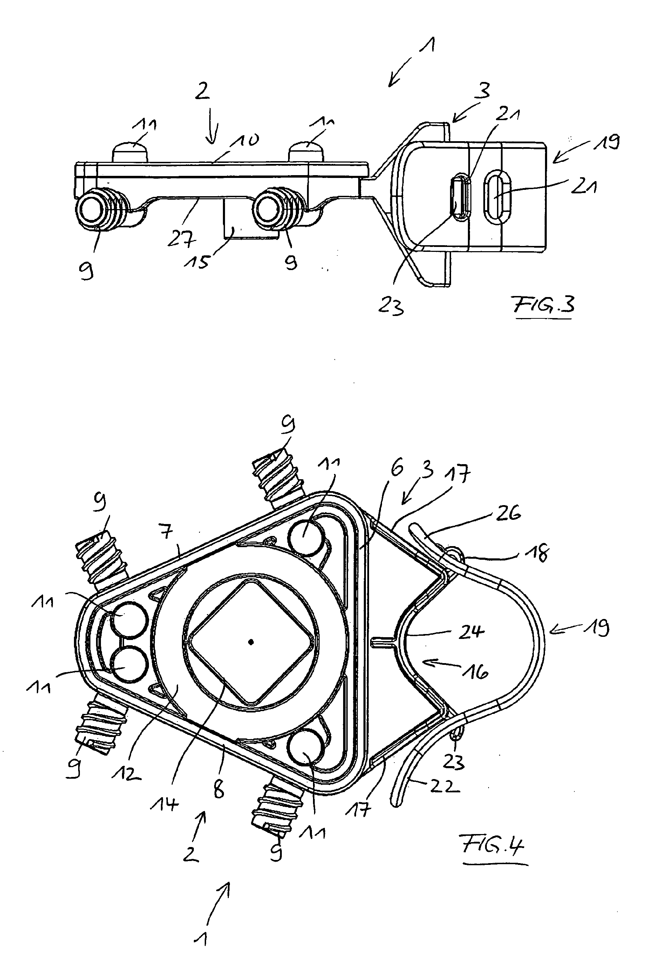

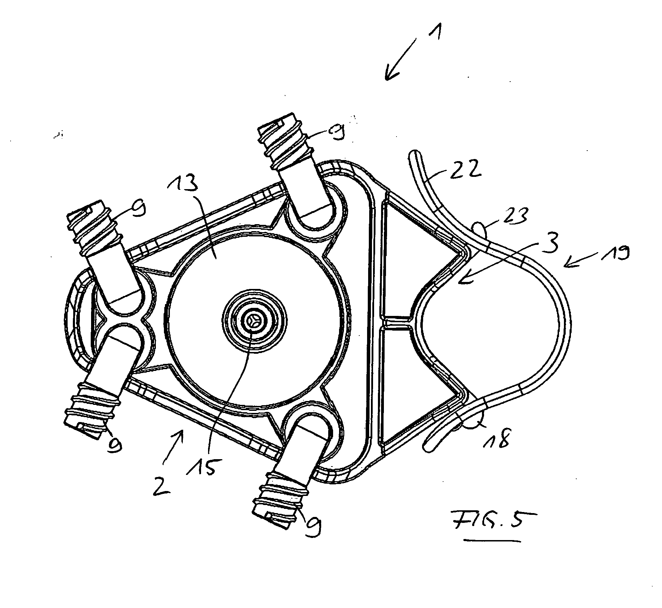

Die in den Figuren 1 bis 5 gezeigte erste Ausführungsform der Vorrichtung 1 zum Zusammenführen mehrerer Infusionen und/oder Injektionen weist ein von der Infusions-/Injektionsflüssigkeit durchströmtes Gehäuses 2, ein innerhalb des Gehäuses 2 angeordnetes, nicht näher veranschaulichtes Mikro-Filterelement für die Flüssigkeit sowie ein mit dem Gehäuse 2 verbundenes Lagerteil 3 auf. Das Gehäuse 2 ist durch ein Gehäuseoberteil 4 und ein Gehäuseunterteil 5 gebildet. In der Draufsicht weist das Gehäuse die Form eines gleichschenkligen Dreiecks auf, mit einer Grundseite 6 und den beiden anderen Seiten 7 und 8 des Dreiecks. Mit der Grundseite 6 ist das Lagerteil 3 verbunden. Im Bereich der Seite 7 weist das Gehäuseunterteil 5 zwei Zugänge 9 und im Bereich der Seite 8 das Gehäuseunterteil 5 zwei weitere Zugänge 9 auf. Die Zugänge 9 sind mit einem Außengewinde versehen, zum Verbinden mit einem Infusionsschlauch oder einem Schlauch zum Injizieren einer medizinischen Flüssigkeit. Die Verbindung zwischen Schlauch und Zugang 9 erfolgt mittels einer Luer-Lock-Kupplung. Die Längsachse der Zugänge 9 verläuft parallel zur oberen Fläche 10 des Gehäuses 2. Die oben hervorragenden kegelstumpfförmigen Ansätze 11, die benachbart des jeweiligen Zuganges 9 angeordnet sind und unterschiedlich farbig sind, dienen der schnellen Identifikation der Zugänge 9 durch das mit der Vorrichtung arbeitende Personal.

Durch die Zugänge 9 wird Infusions-/Injektionsflüssigkeit dem Gehäuseinneren zugeführt, wobei jeder Zugang 9 separat verschließbar ist, insbesondere jeder Zugang 9 ein Rückschlagventil aufweist, das ein Rückströmen der Flüssigkeit verhindert. Die Flüssigkeit gelangt im Gehäuseinneren, ausgehend von den Zugängen 9, in einen Ringraum, der durch die äußeren Ringkonturen 12 und 13 im Gehäuseoberteil 4 bzw. Gehäuseunterteil 5 veranschaulicht ist. Vom Ringraum gelangt die Flüssigkeit durch das Filterelement hindurch, wobei bezüglich dieses Filterelementes die Kontur 14 im Gehäuseoberteil 4, die den Gehäusekammerbereich zur Aufnahme des Filterelements bildet, veranschaulicht ist. Von diesem von der Flüssigkeit durchströmten Filterraum strömt die Flüssigkeit aus der Vorrichtung, und zwar durch den einen zentralen Abgang 15 auf der Unterseite 27 der Vorrichtung, der entsprechend dem jeweiligen Zugang 9 mit einem Außengewinde zwecks Luer-Lock-Anschluss versehen ist.

Das Gehäuse 2 und das Lagerteil 3 sind flach ausgebildet, d.h. es ist die Höhe von Gehäuse 2 und Lagerteil 3 wesentlich geringer als die Abmessung der durch Gehäuse 2 und Lagerteil 3 gebildeten Baueinheit in Längen- und Breitenerstreckung der Baueinheit.

Auf der dem Gehäuse 2 abgewandten Seite weist das Lagerteil 3 eine nach innen, somit zum Gehäuse 2 hin gerichtete Aufnahme 16 auf. Diese ist annähernd kreisbogenförmig ausgebildet und erstreckt sich über einen Winkel von etwas mehr als 90°. An diese Aufnahme 16 schließen sich Flankenabschnitte 17 an, die Bestandteil des Lagerteils 3 sind und die Aufnahme 16 mit dem Gehäuseunterteil 5 im Bereich des Übergangs der Grundseite 6 zu den Seiten 7 und 8 verbinden. Im Übergang von der Aufnahme 6 zum jeweiligen Flankenabschnitt 17 ist der jeweilige Flankenabschnitt 17 mit einem von der Aufnahme weg gerichteten Haken 18 bzw. 23 versehen. Die beiden Haken 18 und 23 dienen dem Einhängen eines elastischen Bandes 19, insbesondere eines Gummibandes, das im Bereich des einen Endes ein Loch 20 und in Abstand zum anderen Ende des Gummibandes 19 zwei in Längsrichtung des Bandes hintereinander angeordnete Löcher 21 aufweist. Das Loch 20 ist so bemessen, dass es bei der Montage der Vorrichtung nur mit erheblichem Kraftaufwand auf den dieser zugeordneten Haken 18 aufgesteckt werden kann und demnach das Band 19 in diesem Bereich bei üblichem Gebrauch der Vorrichtung nicht lösbar ist. Die beiden anderen Löcher 21 hingegen weisen einen Querschnitt auf, der es erlaubt, das Gummiband 19 im Bereich dieser Löcher 21 einfach auf den diesem Ende des Gummibandes 19 zugewandten Haken 23 aufzustecken und durch Ergreifen des Endes 22 des Gummibandes 19 auch wieder von zugeordneten Haken 23 zu lösen.

Die erfindungsgemäße Vorrichtung dient nicht nur dem Zusammenführen der mehreren Infusionen und/oder Injektionen, sondern weist zudem den erörterten Schnellverschluss zum Befestigen der Vorrichtung an einem nicht veranschaulichten Lagerstab eines Standardstativs, das auf dem medizinischen Gebiet Anwendung findet, Verwendung. Hierzu wird die Vorrichtung, bei mit dem Haken 18 verbundenem Gummiband 19 und nicht mit dem Haken 23 verbundenem Gummiband 19, das in Längserstreckung des dem Haken 18 zugeordneten Flankenabschnittes 17 orientiert ist, mit der senkrecht zur oberen Fläche 10 des Gehäuses 2 verlaufenden Anlagefläche der Aufnahme 16 an den der Geometrie der Aufnahme 16 angepassten, Kreisquerschnitt aufweisenden Lagerstab des Standardstativs angelegt und das Gummiband 19 auf der der Aufnahme 16 abgewandten Seite des Lagerstabs vorbei geführt sowie das Gummiband 19 unter Spannung im Bereich einer seiner beiden Öffnungen 21 auf den Haken 23 aufgesteckt. Damit ist die Vorrichtung schnell und sicher mit dem Standardstativ verbunden und umgehend einsetzbar. Die Zugänge 9 und der Abgang 15 können vor oder nach dem Befestigen der Vorrichtung 1 am Lagerstab des Standardstativs mit den Zugangs- bzw. Abgangsleitungen verbunden werden.

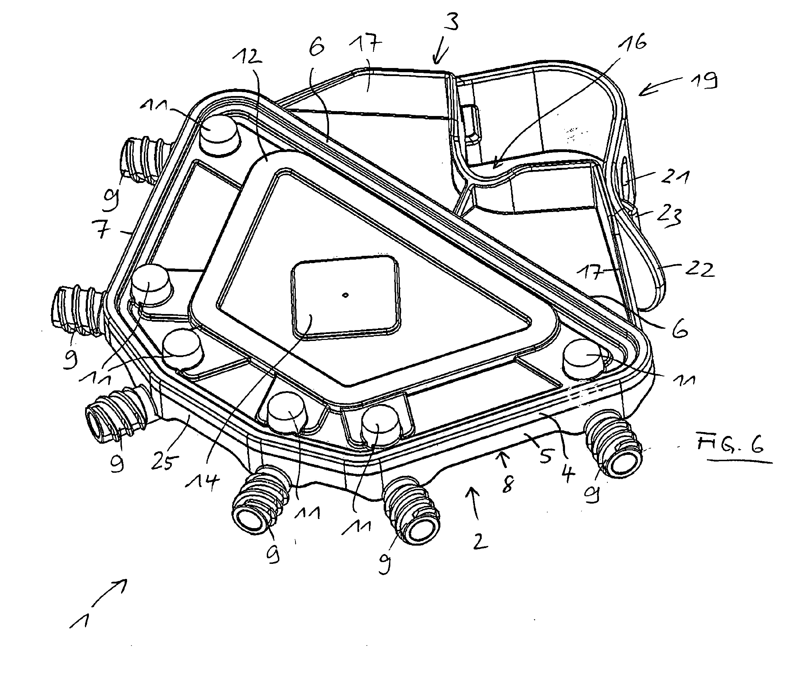

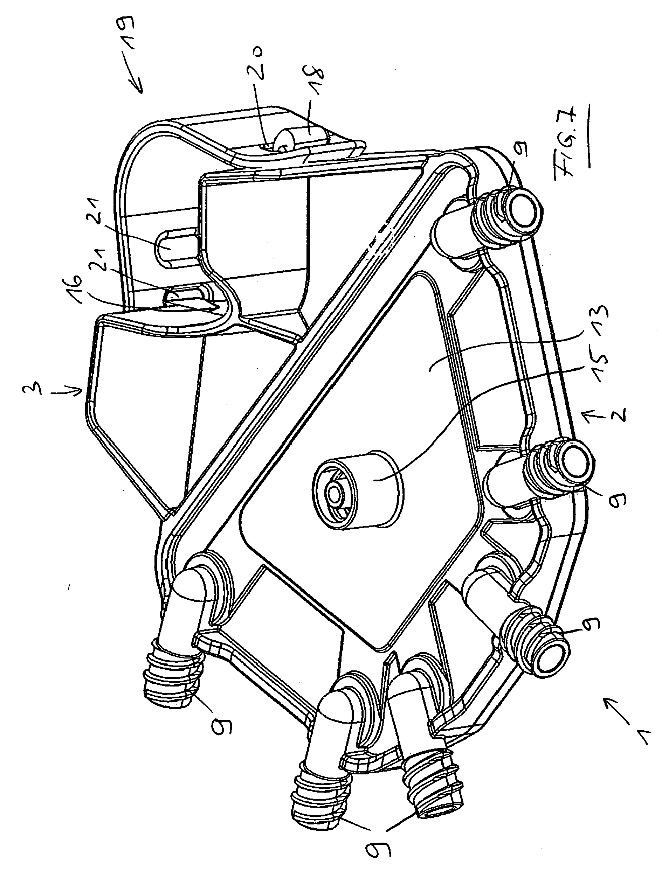

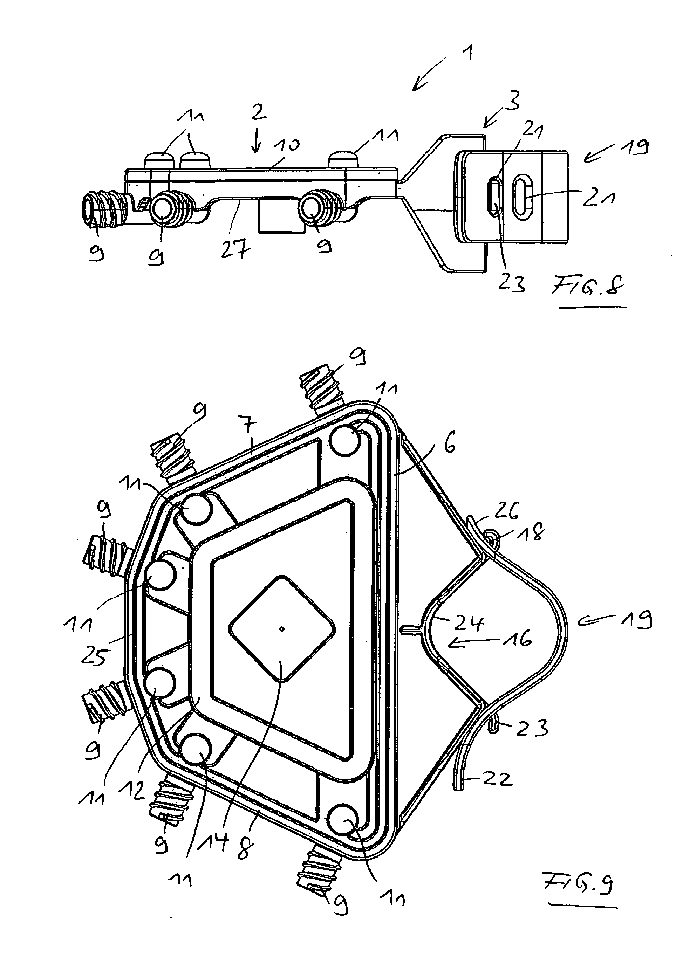

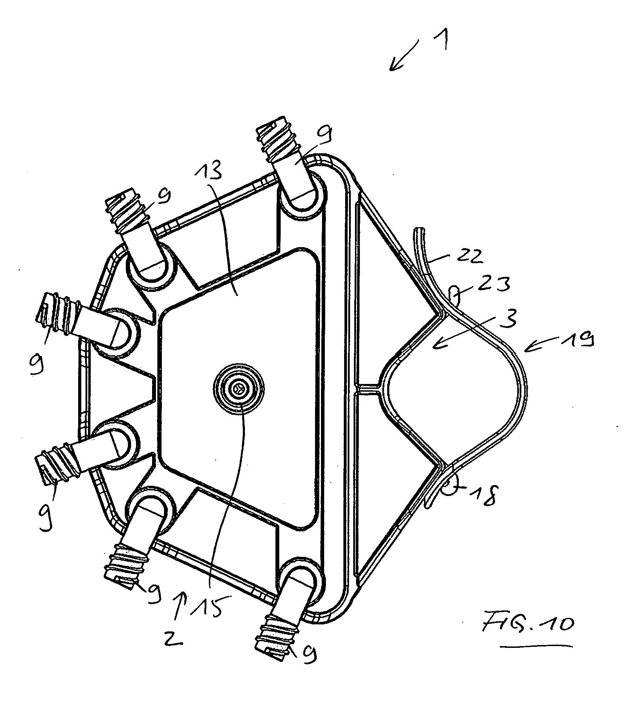

Die Ausführungsform nach den Figuren 6 bis 10 unterscheidet sich von derjenigen nach den Figuren 1 bis 5 nur dadurch, dass statt eines in der Draufsicht dreieckigen Gehäuses 2 ein im wesentlichen trapezförmiges Gehäuse 2 vorgesehen ist. Mit dessen Grundseite 6 ist das Lagerteil 3 verbunden und es schließen sich an die Grundseite 6 die beiden Seiten 7 und 8 mit den jeweils zwei Zugängen an. Die andere Grundseite 25 ist nicht streng parallel 6 angeordnet, sondern weist zwei geringfügige Abwinklungen auf. Im Bereich der beiden Abwinklungen sind zwei weitere Zugänge 9 angeordnet. Das Gehäuseoberteil 4 weist entsprechend den sechs Zugängen 9 sechs unterschiedlich farbig markierte Ansätze 11 benachbart den Zugängen 9 auf. Entsprechend der grundsätzlich trapezförmig gestalteten oberen Fläche 10 des Gehäuses 2 sind die Ringkonturen 12 und 13 als gleichschenkliges Trapez gestaltet. Wegen der größeren Länge der Grundseite 6 des Trapezes gegenüber der Grundseite 6 des Dreiecks nach der Ausführungsform der Figuren 1 bis 5 ist bei der Ausführungsform nach den Figuren 6 bis 10 das Lagerteil 13 länger gestaltet, so dass es sich bis zu den Endbereichen der Grundseite 6 erstreckt.

Im übrigen gelten betreffend die Ausführungsformen nach den Figuren 6 bis 10 die Ausführungen zu der Ausführungsform nach den Figuren 1 bis 5. Die in ihrem Aufbau und/oder in ihrer Funktion übereinstimmenden Teile der beiden Ausführungsformen sind mit denselben Bezugszahlen bezeichnet.

Das Befestigungsmittel kann unterschiedlich gestaltet sein, sofern es den erfindungsgemäßen Zweck erfüllt, das mit dem Gehäuse verbundene Lagerteil sicher am Lagerstab zu befestigen. Neben der bevorzugten Ausbildung des Befestigungsmittels als elastisches Befestigungsmittel ist beispielsweise eine Ausbildung des Befestigungsmittels in Art eines Klettbandes denkbar, das mit komplementären Bandabschnitten mit Hakenteilen zusammenwirkt, die am Lagerteil angebracht sind oder umgekehrt, d.h. am Lagerteil sind Klettbänder angebracht und es ist das Befestigungsmittel ein Hakenband.

## Patentansprüche

1. Vorrichtung (1) zum Zusammenführen mehrerer Infusionen und/oder Injektionen, mit einem von der Infusions-/oder Injektionsflüssigkeit durchströmten Gehäuse (2), sowie mit einem mit dem Gehäuse (2) verbundenen Lagerteil (3), das im Bereich einer Seite (6) des Gehäuses (2) angeordnet ist, und das Gehäuse (2) im Bereich anderer Gehäuseseiten (7, 8, 27; 7, 8, 25, 27) mehrere, jeweils separat verschließbare Zugänge (9) für die Flüssigkeit und einen Abgang (15) für die zusammengeführte Flüssigkeit aufweist, **dadurch gekennzeichnet, dass** das Lagerteil (3) eine nach innen gerichtete Aufnahme (16) aufweist, die dem teilweisen Umschließen eines Lagerstabes für die Vorrichtung (1) dient, sowie ein bandförmiges Befestigungsmittel (19) dem Umschließen der dem Lagerteil (3) abgewandten Seite des Lagerstabs dient, wobei das Befestigungsmittel (19) im Bereich eines Endes (26) mit dem Lagerteil (3) verbunden oder verbindbar ist und das Befestigungsmittel (19) im Bereich des anderen Endes (22) mit dem Lagerteil (3) verbindbar ist.

2. Vorrichtung nach Anspruch 1, **dadurch gekennzeichnet, dass** das Befestigungsmittel (19) elastisch ist.

3. Vorrichtung nach Anspruch 1 oder 2, **dadurch gekennzeichnet, dass** die nach innen gerichtete Aufnahme (16) des Lagerteils (3) bogenförmig ist, insbesondere annähernd kreisbogenförmig ist.

4. Vorrichtung nach einem der Ansprüche 1 bis 3, **dadurch gekennzeichnet, dass** das Befestigungsmittel (19) im Bereich eines Endes (26) fest mit dem Lagerteil (3) verbunden ist.

5. Vorrichtung nach einem der Ansprüche 1 bis 4, **dadurch gekennzeichnet, dass** das Befestigungsmittel (19) zumindest im Bereich eines Endes (26 bzw. 22) ein Loch (20 bzw. 21) aufweist, sowie das Lagerteil (3) in seinem mit diesem Ende (26 bzw. 22) des Befestigungsmittels (19) zusammenwirkenden Bereich mit einem Vorsprung (18 bzw. 23) versehen ist, wobei bei mit dem Lagerteil (3) befestigtem, den Lagerstab umschließenden Befestigungsmittel (19) der Vorsprung (18 bzw. 23) das Loch (20 bzw. 21) durchsetzt und das Befestigungsmittel (19) vorgespannt ist.

6. Vorrichtung nach einem der Ansprüche 3 bis 5, **dadurch gekennzeichnet, dass** das Band (19) im Bereich eines Endes (22) mehrere, insbesondere zwei in Längsrichtung des Bandes (19) hintereinander angeordnete Löcher (21) aufweist.

7. Vorrichtung nach einem der Ansprüche 1 bis 6, **dadurch gekennzeichnet, dass** das Gehäuse (2) flach ausgebildet ist.

8. Vorrichtung nach einem der Ansprüche 1 bis 7, **dadurch gekennzeichnet, dass** das Gehäuse (2) im wesentlichen die Form eines gleichschenkligen Dreiecks oder gleichschenkligen Trapezes aufweist, wobei das Lagerteil (3) im Bereich der Grundseite (6) von Dreieck bzw. Trapez angeordnet ist.

9. Vorrichtung nach Anspruch 8, **dadurch gekennzeichnet, dass** die Zugänge (9) im Bereich der anderen Seiten (7, 8) des Dreiecks bzw. der anderen Seiten (7, 8, 25) des Trapezes angeordnet sind.

10. Vorrichtung nach Anspruch 9, **dadurch gekennzeichnet, dass** bei Ausbildung des Gehäuses (2) als Dreieck die Zugänge (9) im Bereich von zweiten Seiten (7, 8) des Dreiecks, insbesondere im Bereich jeder Seite (7, 8) zwei Zugänge (9) und/oder bei Ausbildung des Gehäuses (2) als Trapez die Zugänge (9) im Bereich von drei Seiten (7, 8, 25) des Trapezes, insbesondere im Bereich jeder Seite (7, 8, 25) zwei Zugänge (9) angeordnet sind.

11. Vorrichtung nach einem der Ansprüche 1 bis 10, **dadurch gekennzeichnet, dass** der Abgang (15) auf der Unterseite (27) des Gehäuses (2) angeordnet ist.

12. Vorrichtung nach einem der Ansprüche 1 bis 11, **dadurch gekennzeichnet, dass** das Gehäuse (2) zumindest zweiteilig ausgebildet ist und ein oberes Gehäuseteil (4) und ein unteres Gehäuseteil (5) aufweist, wobei eines der Gehäuseteile (5) und das Lagerteil (3) einteilig sind.

13. Vorrichtung nach einem der Ansprüche 1 bis 12, **dadurch gekennzeichnet, dass** das Gehäuse (2) und das Lagerteil (3) aus Kunststoff bestehen und als Spritzgussteil ausgebildet sind.

14. Vorrichtung nach einem der Ansprüche 1 bis 13, **dadurch gekennzeichnet, dass** das Gehäuse (2) ein Filterelement für die Flüssigkeit und/oder ein Filterelement für im Gehäuse (2) befindliche Luft aufnimmt.
